# EUROPÄISCHE PATENTSCHRIFT

(11) **EP 3 991 612 B1**
(45) Veröffentlichungstag und Bekanntmachung des Hinweises auf die Patenterteilung: **21.02.2024**
(21) Anmeldenummer: 21152153.9
(22) Anmeldetag: 18.01.2021
(51) Int. Cl.: A47C 27/15

(54) **VIBROAKUSTISCHE EINRICHTUNG ZUR INSBESONDERE MENSCHLICHEN KLANG- UND/ODER VIBRATIONSBEHANDLUNG**
VIBRO-ACOUSTIC DEVICE, IN PARTICULAR FOR HUMAN SOUND AND / OR VIBRATION PROCESSING
DISPOSITIF VIBROACOUSTIQUE, EN PARTICULIER DESTINÉ AU TRAITEMENT DES SONS ET/OU DES VIBRATIONS HUMAINS

(30) Priorität: 28.10.2020 DE 202020106172 U
(43) Veröffentlichungstag der Anmeldung: 04.05.2022
(73) Patentinhaber: Päßler, Michael, 59425 Unna (DE)
(72) Erfinder: Päßler, Michael, 59425 Unna (DE)
(74) Vertreter: Andrejewski - Honke Patent- und Rechtsanwälte Partnerschaft mbB

(56) Entgegenhaltungen:
- EP-A1- 1 683 446
- EP-B1- 1 683 446
- WO-A1-90/04379
- WO-A1-2015/052376
- WO-A1-2019/230304
- CN-U- 204 838 781
- KR-B1- 101 418 944
- US-A- 4 354 067

## Beschreibung

Die Erfindung betrifft eine vibroakustische Einrichtung zur menschlichen Klang- und/oder Vibrationsbehandlung, vorzugsweise eine Klangmatte, mit wenigstens einer flexiblen Grundplatte sowie einer mehrere Körperschallwandler aufweisenden Schallerzeugungseinheit, wobei der jeweilige Körperschallwandler in die Grundplatte eingebettet ist, um den erzeugten Körperschall direkt in wenigstens ein Körperteil einzukoppeln, wobei ferner die Schallerzeugungseinheit wenigstens ein Steuergerät aufweist, und wobei das Steuergerät zur Ansteuerung des jeweiligen Körperschallwandlers ein konstantes sowie gegebenenfalls einstellbares niederfrequentes Körperschallsignal zur direkten Einkopplung in das betreffende Körperteil erzeugt.

Die fragliche vibroakustische Einrichtung wird in der Regel zur Behandlung von Menschen mit Klang bzw. Vibrationen eingesetzt. Grundsätzlich können an dieser Stelle aber auch Tiere und insbesondere Säugetiere einer solchen Behandlung unterzogen werden. Hier kommen typischerweise domestizierte Tiere in Frage, wie beispielsweise Pferde, Hunde, Katzen usw. Außerdem kann die Klangmatte nicht nur zur Behandlung im Liegen, sondern auch im Sitzen bei und in Verbindung mit Rollstuhlfahrern, Senioren etc. zum Einsatz kommen. D.h., der Begriff Klangmatte ist weit dahingehend zu verstehen und auszulegen, dass hiermit nicht nur Liegematten, sondern beispielsweise auch Sitzkissen, Sitzmatten etc. abgedeckt werden.

Solche vibroakustischen Einrichtungen, wie sie beispielsweise in der DE 20 2014 003 867 U1 beschrieben werden, kommen typischerweise zum Einsatz, um im Innern des menschlichen Körpers Körperschallwellen zu erzeugen. Die Körperschallwellen erfassen dabei den gesamten Körper oder einzelne Bereiche bzw. Körperteile. Als Folge dieser den Körper durchströmenden Körperschallwellen kommt es zur gezielten Massage der jeweils angesprochenen Bereiche. Das zieht meistens eine nachhaltige Entspannung der Muskulatur und eine Erhöhung der Durchblutung nach sich und wird von behandelten Personen als überaus angenehm empfunden.

Zu diesem Zweck kommt im Stand der Technik nach der DE 20 2014 003 867 U1 eine Behandlungsliege zum Tragen, die mit einem Resonanzsystem ausgerüstet ist. An der Unterseite der Behandlungsliege sind auf ihrer Bodenplatte ein oder mehrere Schallwandler angeordnet, die in einem Frequenzbereich von 20 bis 200 Hz arbeiten. Die Schallwandler können dabei auch mit einem Audiosignal beaufschlagt werden. Außerdem ist die Stärke der Vibration der Schallwandler regelbar.

Durch die US 2020/0294407 A1 ist eine Klangmatte für Kleinkinder bekannt geworden, die einen eingebauten Lautsprecher aufweist. Der Lautsprecher kann mit Audiosignalen angesprochen werden und gibt entsprechende Musiksignale über Perforationen in der Matte nach außen hin ab.

Körperschallwandler bzw. Körperschallerzeuger, bei denen es sich grundsätzlich um Lautsprecher ohne Lautsprechermembran handelt, kommen auch auf Nachbargebieten zum Einsatz. So ist durch die DE 10 2012 113 034 B3 eine Sanitärwannenanordnung mit einem Schallerzeugungssystem bekannt geworden. Dabei ist ein an einen Wannenkörper angeschlossener Körperschallwandler realisiert. Der Körperschallwandler ist an einer stoffschlüssig mit dem Wannenkörper verbundenen und von einer Außenwand des Wannenkörpers abstehenden Lasche befestigt. Dadurch wird eine Schallübertragung von dem Körperschallwandler auf den Wannenkörper bewirkt.

Im Rahmen der DE 10 2007 010 247 B3 geht es um ein Wasserbett, welches über einen am Boden stehenden Sockel und eine auf dem Sockel abgestützte Bodenplatte verfügt. Außerdem liegt auf der Bodenplatte eine Wassermatratze auf. An der Unterseite der Bodenplatte ist ein Körperschallwandler schallübertragend befestigt. Dadurch können einfach und kostengünstig Schwingungen des Schallschwingungsgebers in die Wassermatratze eingeleitet werden.

Durch die EP 1 683 444 A1 ist eine gattungsgemäße vibroakustische Einrichtung bekannt geworden. Dabei kommt als Matte ein Körper aus Kunststoff mit einer Federstruktur zum Einsatz. In den fraglichen Körper sind mehrere Lautsprecher eingebettet.

Der Stand der Technik hat sich grundsätzlich bewährt, geht allerdings typischerweise so vor, dass der vom Körperschallwandler erzeugte Körperschall über mehr oder minder voluminöse sowie zwischengeschaltete Materialschichten in den Körper eingekoppelt wird. Tatsächlich ist bei der Lehre nach der DE 20 2014 003 867 U1 die Unterseite der Behandlungsliege mit einer Bodenplatte ausgerüstet, die wiederum den angeschlossenen Schallwandler oder Körperschallwandler aufweist. Dadurch werden die vom Körperschallwandler erzeugten Vibrationen über die Bodenplatte, den meistens flexiblen Kern der Behandlungsliege schließlich in den Körper des Probanden eingekoppelt. Vergleichbar geht die US 2020/0294407 A1 vor, wo an dieser Stelle nicht ein Körperschallwandler sondern ein herkömmlicher Lautsprecher zum Einsatz kommt, der die Schallwellen durch Luftübertragung bis hin zum Kleinkind weiterleitet. Bei dem Wasserbett nach der DE 10 2007 010 247 B3 erfolgt die Schallübertragung vom Körperschallwandler über eine Deckplatte auf das eigentliche Wasserbett, d.h., unter Zwischenschaltung von Wasser. Ähnliches gilt für die Sanitärwannenanordnung entsprechend der DE 10 2012 113 034 B3, wo zwischen dem am Wannenkörper angebrachten Körperschallwandler und dem menschlichen Körper wiederum Wasser zur Schallübertragung zwischengeschaltet ist. Als Folge dieser gleichsam indirekten Körperschallübertragung ist die Wirkung der bekannten Körperschallwandler begrenzt und insbesondere ihre Amplitude vermindert. Außerdem kommt es durch die zwischengeschalteten Materialien oder Flüssigkeiten dazu, dass eine diffuse Schallausbreitung beobachtet wird. Hier will die Erfindung insgesamt Abhilfe schaffen.

WO 90/04379 A1 offenbart eine Vorrichtung nach dem Oberbegriff des Anspruchs 1.

### ZUSAMMENFASSUNG DER ERFINDUNG

Die Erfindung ist in den beigefügten Ansprüchen wiedergegeben.

### ZUSAMMENFASSUNG DER OFFENBARUNG

Der Erfindung liegt das technische Problem zugrunde, eine derartige vibroakustische Einrichtung so weiterzuentwickeln, dass eine besonders wirkungsvolle Einkopplung des Körperschalls in den menschlichen Körper gelingt und darüber hinaus die Möglichkeit besteht, gezielt einzelne Körperregionen ansprechen zu können.

Zur Lösung dieser technischen Problemstellung ist eine gattungsgemäße vibroakustische Einrichtung zur menschlichen Klang- und/oder Vibrationsbehandlung im Rahmen der Erfindung dadurch gekennzeichnet, dass das niederfrequente Körperschallsignal mit einem demgegenüber höherfrequenten Audiosignal überlagert ist, und dass mithilfe des Steuergerätes die Frequenz und Amplitude sowie Zeitdauer des Körperschallsignals und des Audiosignals des betreffenden Körperschallwandlers jeweils einstellbar sind, wobei mithilfe des Steuergerätes einzelne ausgewählte Körperschallwandler und/oder Gruppen von Körperschallwandlern unterschiedlich ansteuerbar sind.

Bei der flexiblen Grundplatte handelt es sich in der Regel um eine solche, die aus zwei gleichgroßen Teilplatten zusammengesetzt ist, die zwischen sich die Körperschallwandler aufnehmen. Dabei wird meistens so vorgegangen, dass der jeweilige Körperschallwandler mit seiner Montageplatte an eine Unterseite der oberen Teilplatte der Grundplatte angeschlossen ist. Tatsächlich setzt sich der Körperschallwandler aus den wesentlichen drei Komponenten, nämlich der schwingfähigen Masse, einem Anschlussterminal zur elektrischen Verbindung und der zuvor bereits angesprochenen Montageplatte zusammen. Über das Anschlussterminal kann die notwendige elektrische Verbindung mit einem Verstärker bzw. einem Steuergerät hergestellt werden, um das vibroakustische Signal einzuspeisen. Als Folge hiervon vibriert die schwingfähige Masse im Takt des eingespeisten Signals.

Bei dem fraglichen Signal handelt es sich erfindungsgemäß um ein niederfrequentes Körperschallsignal, welches mit dem demgegenüber höherfrequenten Audiosignal überlagert wird. Das niederfrequente Körperschallsignal erzeugt typischerweise Vibrationen im Bereich zwischen 20 Hz und 500 Hz oder noch mehr, damit die auf diese Weise erzeugten Körperschallwellen direkt und unmittelbar in den menschlichen Körper übertragen werden können. Hierbei geht die Erfindung von der Erkenntnis aus, dass solche Körperschallwellen vom größtenteils Wasser beinhaltenden Körper besonders wirksam aufgenommen und weitergeleitet werden können. Demgegenüber dient das gegebenenfalls überlagerte höherfrequente Audiosignal in der Regel dazu, zusätzlich beispielsweise eine Melodie zu erzeugen, die von der zu behandelnden Person akustisch per Luftwellen und über die Ohren aufgenommen wird.

Die niederfrequenten Schwingungen des Körperschallsignals werden von der schwingfähigen Masse auf die Montageplatte übertragen. Da die Montageplatte an die Unterseite der oberen Teilplatte der Grundplatte angeschlossen ist und die Grundplatte flexibel ausgelegt ist, werden die betreffenden Vibrationen der Montageplatte gleichsam direkt auf den menschlichen Körper übertragen. Denn die flexible Grundplatte ist in der Regel als Bestandteil der Klangmatte ausgebildet, so dass die zu behandelnde Person auf der fraglichen Klangmatte bzw. der Grundplatte aufliegt.

Da die Grundplatte flexibel ausgebildet ist und die Körperschallwandler zwischen den beiden die Grundplatte bildenden Teilplatten angeordnet sind, wird zumindest die obere Teilplatte durch den aufliegenden Körper so weit komprimiert, dass über die betreffenden Körperschallwandler das von ihnen erzeugte Körperschallsignal direkt in den Körper des zu behandelnden Menschen eingekoppelt werden kann. Hierbei geht die Erfindung von der weiteren Erkenntnis aus, dass die beiden Teilplatten typischerweise aus einem Schaumkunststoff hergestellt sind, der beispielhaft eine Stärke zwischen 2 cm und 5 cm aufweist. Solche Schaumkunststoffe werden folglich beim Aufliegen des Körpers des zu behandelnden Menschen auf der Grundplatte soweit komprimiert, dass der jeweilige Körperschallwandler und der hierdurch erzeugte Körperschall direkt in das darüber befindliche Körperteil des Probanden eingekoppelt wird. Zugleich sorgt die untere Teilplatte dafür, dass insgesamt der Liegekomfort nicht beeinträchtigt wird und die zu behandelnde Person die Behandlung nicht als unangenehm empfindet.

Wie bereits erläutert, ist der jeweilige Körperschallwandler mit seiner Montageplatte an die Unterseite der oberen Teilplatte der Grundplatte angeschlossen. Darüber hinaus ist die Auslegung erfindungsgemäß so getroffen, dass der jeweilige Körperschallwandler mit seiner schwingfähigen Masse nach außen weisend (in Bezug auf die obere Teilplatte) auf der Oberseite der unteren Teilplatte angeordnet ist. Dadurch kann die schwingfähige Masse in die ebenfalls flexibel ausgebildete untere Teilplatte eintauchen, sobald der Körper einer zu behandelnden Person auf der Grundplatte aufliegt. Meistens wird hier zusätzlich noch so vorgegangen, dass der an die obere Teilplatte angeschlossene jeweilige Körperschallwandler mit einer textilen Flächenschicht abgedeckt ist. Bei der textilen Flächenschicht kann es sich um eine solche handeln, die beispielsweise aus einer Watte oder einem Vlies oder auch einem Gewebe hergestellt ist. Mit Hilfe der textilien Flächenschicht wird der jeweilige Körperschallwandler gegenüber der unteren Teilplatte abgedeckt und werden zugleich die einzelnen zum Anschlussterminal des jeweiligen Körperschallwandlers hin führenden Zuleitungskabel geschützt. D.h., die die Körperschallwandler an der Unterseite der oberen Teilplatte tragende obere Teilplatte wird mit Hilfe der textilen Flächenschicht praktisch komplett zur unteren Teilplatte hin abgedeckt und verschlossen. Hierfür sorgt die textile Flächenschicht.

So oder so sorgt die Vibration der betreffenden Montageplatte des Körperschallwandlers zur Erzeugung des gewünschten Körperschalls im niederfrequenten Bereich zwischen typischerweise 20 Hz und 500 Hz oder sogar noch mehr.

Der Rückgriff auf Körperschallwandler an dieser Stelle ist dabei mit dem weiteren Vorteil verbunden, dass die Schallabstrahlung der massiven Montageplatte im Vergleich zu einer demgegenüber viel leichteren Lautsprechermembran anders erfolgt. Tatsächlich breiten sich sogenannte Biegewellen auf der zu Schwingungen angeregten Montageplatte aus, die durch die erfindungsgemäße Anordnung unmittelbar als Körperschall in den menschlichen Körper des Probanden eindringen können. Dadurch entstehen insgesamt auf der Montageplatte wellenförmige Schwingungen vergleichbar der Wellenausbreitung bei einem in ein Wasserbecken geworfenen Stein. Diese wellenförmigen Schwingungen sind aufgrund der meistens quadratischen Auslegung der Montageplatte im Zentrum dieser Montageplatte konzentriert bzw. gehen hiervon aus, so dass mit Hilfe des betreffenden Körperschallwandlers bzw. Körperschallerzeugers der zugehörige Körperteil gezielt angesprochen werden kann. Da zwischen der Montageplatte und dem Körperteil lediglich die durch das Körpergewicht komprimierte obere Teilplatte angeordnet ist (ggf. in Verbindung mit einem dünnen Überzug) gelingt die Schalleinkopplung unmittelbar und gezielt.

Die Schallerzeugungseinheit weist in der Regel nicht nur die mehreren Körperschallwandler auf, sondern meistens ist zusätzlich noch wenigstens ein Steuergerät als Bestandteil der Schallerzeugungseinheit vorgesehen. Das Steuergerät dient zur Ansteuerung des jeweiligen Körperschallwandlers mit einem konstanten sowie gegebenenfalls einstellbaren niederfrequenten Körperschallsignal. Über das Körperschallsignal wird der mit Hilfe des Körperschallwandlers erzeugte Körperschall direkt in den betreffenden Körperteil eingekoppelt. Dabei wird meistens und je nach Körperteil mit unterschiedlichen sowie regelmäßig konstanten niederfrequenten Körperschallsignalen gearbeitet.

Das demgegenüber dem niederfrequenten Körperschallsignal überlagerte höherfrequente Audiosignal verfügt über eine wechselnde Frequenz, die beispielsweise als Musik- oder Sprachuntermalung oder beides vom zu behandelnden Probanden wahrgenommen wird. Mit Hilfe des Steuergerätes werden dabei die Frequenz und/oder Amplitude sowie Zeitdauer des Körperschallsignals und des Audiosignals jeweils eingestellt. Die Einstellung kann dabei von Körperschallwandler zu Körperschallwandler unterschiedlich vorgenommen werden. Auf diese Weise lassen sich mit Hilfe des Steuergerätes einzelne ausgewählte Körperschallwandler und/oder Gruppen von Körperschallwandlern unterschiedlich ansteuern.

Darüber hinaus wird meistens so vorgegangen, dass die Körperschallwandler gleichmäßig verteilt über die Grundplatte in ihrem Innern angeordnet sind. Dabei wird man die Anordnung der Körperschallwandler typischerweise so vornehmen, dass die Körperschallwandler insgesamt ein Flächenprofil abdecken, welches die Anregung sämtlicher Körperteile der zu behandelnden Person ermöglicht. Hierzu gehören nicht nur die beiden Arme und Beine, sondern beispielfalls auch der Brust- und Beckenbereich ebenso wie der Kopf. Jedenfalls trägt die Anordnung der Körperschallwandler im Innern der flexiblen Grundplatte dieser gleichsam genormten Silhouette des Körpers eines Probanden Rechnung.

Außerdem ist die Auslegung meistens so getroffen, dass die Grundplatte in zumindest zwei Segmente mit jeweils eigenen Körperschallwandlern unterteilt ist. Dabei können die Körperschallwandler in den beiden Segmenten unterschiedlich angesteuert werden. Meistens sind die beiden Segmente etwa gleich groß ausgebildet und können beispielhaft als Oberkörpersegment und Unterkörpersegment ausgebildet sein. Dadurch lässt sich der Oberkörper mit den zugehörigen Körperschallwandlern anders als der Unterkörper ansteuern. Grundsätzlich ist natürlich auch eine unterschiedliche Ansteuerung beispielsweise nach rechter oder linker Körperhälfte möglich und wird von der Erfindung mit abgedeckt.

Im Ergebnis wird eine vibroakustische Einrichtung zur menschlichen Klang- und/oder Vibrationsbehandlung zur Verfügung gestellt, die die gezielte Einkopplung von Körperschallwellen in den menschlichen Körper bewirkt und zur Verfügung stellt, und zwar mit einer im Vergleich zum Stand der Technik nicht nur gesteigerten Intensität, sondern auch bisher ungewöhnlicher Richtungscharakteristik. Das lässt sich darauf zurückführen, dass der jeweilige Körperschallwandler in die Grundplatte eingebettet ist und dadurch der von ihm erzeugte Körperschall direkt in das mit dem Körperschallwandler in Kontakt kommende Körperteil der zu behandelnden Person eingekoppelt werden kann. Da darüber hinaus die gleichsam mit dem betreffenden Körperteil in Körperschallkontakt kommende Montageplatte Biegewellen erzeugt, deren Zentrum aufgrund der massiven Ausbildung der Montageplatte in quadratischer Form und meistens aus Stahl mit dem Zentrum der Montageplatte zusammenfällt, lassen sich die hierdurch erzeugten Körperschallwellen konzentriert in das jeweilige Körperteil einkoppeln. Etwaige großvolumige und zwischengeschaltete Materialschichten müssen dabei nicht passiert werden.

Durch die zusätzliche Möglichkeit, die einzelnen Körperschallwandler praktisch separat ansprechen zu können oder doch zumindest das Oberkörpersegment getrennt vom Unterkörpersegment, lassen sich die einzelnen Körperregionen der zu behandelnden Person individuell mit Körperschall behandeln. Dadurch werden bisher nicht für möglich gehaltene Behandlungserfolge insbesondere im Hinblick auf die Entspannung der jeweiligen Muskulatur und die Anregung der Durchblutung beobachtet. Hierin sind die wesentlichen Vorteile zu sehen.

Im Folgenden wird die Erfindung anhand einer lediglich ein Ausführungsbeispiel darstellenden Zeichnung näher erläutert; es zeigen:
- Figur 1: der öffnungsgemäße vibroakustische Einrichtung in einer Übersicht und
- Figur 2: den Gegenstand nach der Figur 1 im Querschnitt.

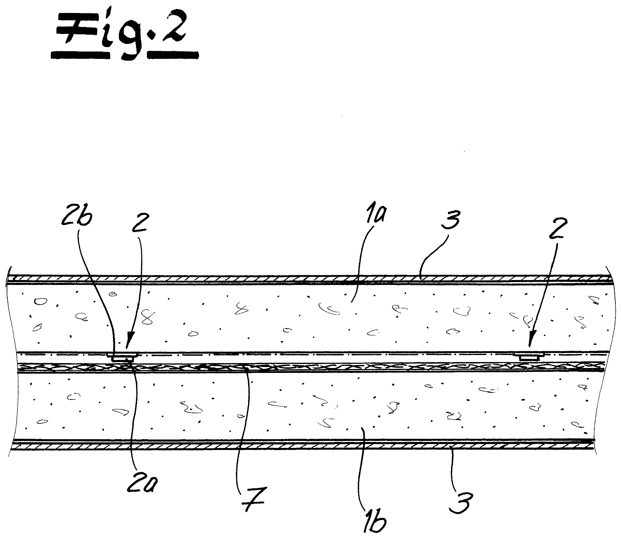

In den Figuren ist eine vibroakustische Einrichtung zur menschlichen Klang- und/oder Vibrationsbehandlung dargestellt, im Ausführungsbeispiel eine sogenannte Klangmatte. Diese verfügt über wenigstens eine flexible Grundplatte 1a, 1b, die sich ausweislich der Schnittdarstellung nach der Fig. 2 aus einer oberen Teilplatte 1a und einer unteren Teilplatte 1b zusammensetzt. Die beiden Teilplatten 1a, 1b sind jeweils rechteckförmig gestaltet und verfügen typischerweise über eine Materialstärke von mehreren Zentimetern, beispielsweise von 2 cm bis 5 cm. Außerdem sind die beiden Teilplatten 1a, 1b aus einem Schaumkunststoff produziert, so dass sie einerseits eine Anpassung an den Körper der zu behandelnden Person beim Liegen auf der Klangmatte zur Verfügung stellen und andererseits die erfindungsgemäß zwischen den beiden Teilplatten 1a, 1b aufgenommenen und nachträglich noch näher zu beschreibenden Körperschallwandler 2 hierbei keine Abdrücke erzeugen.

Anhand des Schnittaufbaus nach der Fig. 2 erkennt man, dass die Grundplatte 1a, 1b bzw. die beiden Teilplatten 1a, 1b insgesamt von einem Bezug 3 umschlossen werden. Bei dem Bezug 3 kann es sich um ein Kunstleder oder auch einen anderen dauerhaften und pflegeleichten Schutzbezug handeln. Dieser verfügt in der Regel über ein Flächengewicht von ca. 500 g/m² bis 800 g/m² und eine Dicke im Bereich von einigen Millimetern, beispielsweise 1 mm bis 3 mm. Das gilt selbstverständlich nur beispielhaft und ist keineswegs einschränkend.

Von besonderer Bedeutung für die Erfindung sind nun neben der flexiblen Grundplatte 1a, 1b die zuvor bereits angesprochenen und mehreren Körperschallwandler 2, die einen Bestandteil einer Schallerzeugungseinheit 2, 4 darstellen. Tatsächlich setzt sich die Schallerzeugungseinheit 2, 4 nicht nur aus den mehreren Körperschallwandlern 2 sondern auch einem Steuergerät 4 zusammen.

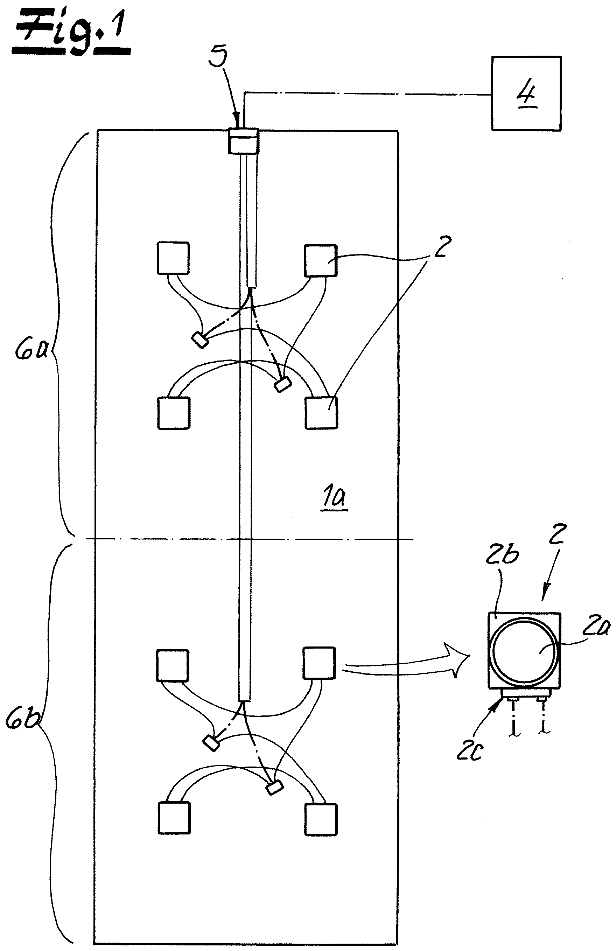

Der Detailaufbau des Körperschallwandlers 2 ist in der vergrößerten Darstellung in der Fig. 1 wiedergegeben. Tatsächlich erkennt man, dass der Körperschallwandler 2 im Wesentlichen aus drei Komponenten zusammengesetzt ist, nämlich einer schwingfähigen Masse 2a, einer Montageplatte 2b und schließlich einem Anschlussterminal 2c. Das Steuergerät 4 ist über entsprechende Zuleitungen mit dem jeweiligen Anschlussterminal 2c des zugehörigen Körperschallwandlers 2 elektrisch verbunden. Dazu kann die dargestellte Klangmatte mit einer Anschlussbuchse ausgerüstet sein, in die ein an das Steuergerät 4 angeschlossener Anschlussstecker 5 eingesteckt werden kann. Bei der Anschlussbuchse bzw. dem Anschlussstecker 5 kann es sich um einen USB-Anschluss handeln, was erneut nur beispielhaft gilt und nicht einschränkend zu verstehen ist.

Mit Hilfe des Steuergerätes 4 können die einzelnen Schallwandler 2 angesteuert werden, und zwar nach dem Ausführungsbeispiel getrennt voneinander und jeweils jeder für sich genommen. Meistens ist die dargestellte Grundplatte 1a, 1b in zumindest zwei Segmente 6a, 6b unterteilt, nämlich ein Oberkörpersegment 6a und ein Unterkörpersegment 6b. Dabei ist die Auslegung insgesamt so getroffen, dass die Körperschallwandler 2 in dem zugehörigen Segment 6a, 6b unterschiedlich angesteuert werden können, so dass auch eine unterschiedliche Ansteuerung des Oberkörpersegmentes 6a im Vergleich zum Unterkörpersegment 6b gelingt.

Von besonderer erfinderischer Bedeutung ist nun der Umstand, dass der jeweilige Körperschallwandler 2 in die Grundplatte 1a, 1b eingebettet ist, um den erzeugten Körperschall direkt in wenigstens ein Körperteil einzukoppeln. Dazu sind die Körperschallwandler 2 zunächst einmal gleichmäßig verteilt über die Grundplatte 1a, 1b in ihrem Innern angeordnet. Tatsächlich wird hier so vorgegangen, dass der jeweilige Körperschallwandler 2 mit seiner Montageplatte 2b an eine Unterseite der oberen Teilplatte 1a der Grundplatte 1a, 1b angeschlossen ist, wie man anhand der Schnittdarstellung in der Fig. 2 erkennen kann. Dazu wird in der Regel die zugehörige Montageplatte 2b des zugehörigen Körperschallwandlers 2 adhäsiv mit der fraglichen Unterseite der oberen Teilplatte 1a verbunden. Dadurch ist der jeweilige Körperschallwandler 2 mit seiner schwingfähigen Masse 2a nach außen weisend auf der Oberseite der unteren Teilplatte 1b angeordnet.

Da die untere Teilplatte 1b ebenso wie die obere Teilplatte 1a jeweils flexibel ausgebildet ist und vorteilhaft aus dem Schaumkunststoff gefertigt wird, dringt die flach zylindrisch ausgebildete schwingfähige Masse 2a in die fragliche untere Teilplatte 1b ein, sobald sich ein Proband auf die Klangmatte legt. Etwaige Abdrücke sind durch die spezifische Auslegung nicht zu erwarten. Vielmehr werden die in Richtung auf den Probanden orientierten jeweiligen Montageplatten 2b des zugehörigen Körperschallwandlers 2 in Anlage an das betreffende Körperteil gebracht, so dass die mit Hilfe des Körperschallwandlers 2 erzeugten Schwingungen direkt in den Körper des zu behandelnden Menschen eingekoppelt werden können.

Anhand der Übersicht nach der Fig. 1 in Verbindung mit der Schnittdarstellung nach der Fig. 2 erkennt man, dass nicht nur der jeweilige Körperschallwandler 2 mit seiner schwingfähigen Masse 2a nach außen weisend auf der Oberseite der unteren Teilplatte 1b angeordnet ist, sondern an dieser Stelle zusätzlich noch eine textile Flächenschicht 7 zwischengeschaltet ist. Mit Hilfe dieser textilen Flächenschicht 7 wird die Unterseite der oberen Teilplatte 1a gleichsam abgedeckt. Dadurch ist sichergestellt, dass die zu den Körperschallwandlern 2 hinführenden Zuleitungen mit Hilfe der textilen Flächenschicht 7 geschützt werden. Gleiches gilt für die Körperschallwandler 2. Bei der textilen Flächenschicht 7 kann es sich um eine solche aus Watte, einem Vlies oder auch einem Gewebe handeln. Typischerweise verfügt die textile Flächenschicht 7 über ein Flächengewicht von 100 g/m² bis 200 g/m² und ist beispielsweise aus Polyesterfasern hergestellt, um eine dauerhafte und abriebfeste Auslegung der textilen Flächenschicht 7 zur Verfügung zu stellen.

Wie bereits erläutert, weist die Schallerzeugungseinheit 2, 4 neben den einzelnen Körperschallwandlern 2 das Steuergerät 4 auf. Die Körperschallwandler 2 sind dabei verteilt über die Grundplatte 1a, 1b in ihrem Innern angeordnet. Die Verteilung der Körperschallwandler 2 trägt dabei einer Silhouette einer auf der Klangmatte liegenden Durchschnittsperson Rechnung. Nach dem Ausführungsbeispiel sind sowohl vier Körperschallwandler 2 für das Oberkörpersegment 6a als auch vier Körperschallwandler 2 für das Unterkörpersegment 6b vorgesehen, was selbstverständlich nur beispielhaft gilt und keinesfalls zwingend ist.

Mit Hilfe des Steuergerätes 4 kann nun der jeweilige Körperschallwandler 2 angesteuert werden. Dabei wird in der Regel ein konstantes sowie gegebenenfalls einstellbares niederfrequentes Körperschallsignal im Frequenzbereich zwischen 20 Hz und 500 Hz oder noch mehr erzeugt. Je nach zu behandelndem Körperteil kann mit unterschiedlichen und fest eingestellten Körperschallsignalen von beispielsweise 50 Hz, 60 Hz oder auch anderen Frequenzen gearbeitet werden. Generell sind natürlich neben festen Frequenzen auch wechselnde Frequenzen denkbar und können mit Hilfe des Steuergerätes 4 bei der Erzeugung des Körperschallsignales vorgegeben werden.

Meistens ist dem niederfrequenten Körperschallsignal im Frequenzbereich zwischen 20 Hz und 500 Hz ein demgegenüber höherfrequentes Audiosignal im Frequenzbereich ab 500 Hz oder 1000 Hz und mehr überlagert. Bei diesem Audiosignal kann es sich um Sprache und/oder Musik handeln, die dem Körperschallsignal meistens mit geringerer Amplitude überlagert wird und von dem Körperschallwandler 2 als Schallwelle abgestrahlt sowie seitens des zu behandelnden Probanden als Musik oder Sprache wahrgenommen wird. Das gilt selbstverständlich nur als Option und ist keinesfalls zwingend.

Das Audiosignal kann dabei generell von dem Steuergerät 4 ebenso wie das Körperschallsignal erzeugt werden. Dazu mag das Steuergerät 4 über beispielsweise eine nicht ausdrücklich dargestellte Leseeinheit verfügen, in die ein Speichermedium eingesteckt wird. Grundsätzlich ist auch eine drahtlose Datenübertragung beispielsweise von einem Computer, einem Mobiltelefon etc. denkbar. Außerdem kann das Steuergerät 4 generell in ein Computernetzwerk eingebunden und hierüber mit den erforderlichen Daten zur Erzeugung des niederfrequenten Körperschallsignals ebenso wie des höherfrequenten Audiosignals versorgt werden.

Darüber hinaus kann mit Hilfe des Steuergerätes 4 die Frequenz und/oder Amplitude und/oder Zeitdauer des Körperschallsignals sowie gegebenenfalls des Audiosignals jeweils eingestellt werden. Das kann für jeden einzelnen Körperschallwandler 2 getrennt mit Hilfe des Steuergerätes 4 vorgenommen werden. Nach dem Ausführungsbeispiel werden jedoch die Körperschallwandler 2 des Oberkörpersegmentes 6a und die Körperschallwandler 2 des Unterkörpersegmentes 6b jeweils gemeinsam mit Hilfe der Steuergerätes 4 angesteuert. Es erfolgt lediglich eine Trennung zwischen den Signalen einerseits für das Oberkörpersegment 6a und andererseits das Unterkörpersegment 6b. D.h., mit Hilfe des Steuergerätes 4 können einzelne ausgewählte Körperschallwandler 2 ebenso wie Gruppen von Körperschallwandler 2 unterschiedlich angesteuert werden.

## Patentansprüche

1. Vibroakustische Einrichtung zur menschlichen Klang- und/oder Vibrationsbehandlung, vorzugsweise Klangmatte, mit wenigstens einer flexiblen Grundplatte (1a, 1b) sowie einer mehrere Körperschallwandler (2) aufweisenden Schallerzeugungseinheit (2, 4), wobei
der jeweilige Körperschallwandler (2) in die Grundplatte (1a, 1b) eingebettet ist, um den erzeugten Körperschall direkt in wenigstens ein Körperteil einzukoppeln, wobei ferner
die Schallerzeugungseinheit (2, 4) wenigstens ein Steuergerät (4) aufweist, und wobei
das Steuergerät (4) zur Ansteuerung des jeweiligen Körperschallwandlers (2) ein konstantes sowie einstellbares niederfrequentes Körperschallsignal zur direkten Einkopplung in das betreffende Körperteil erzeugt, wobei mithilfe des Steuergerätes (4) einzelne ausgewählte Körperschallwandler (2) und/oder Gruppen von Körperschallwandlern (2) unterschiedlich ansteuerbar sind,
**dadurch gekennzeichnet, dass**
das niederfrequente Körperschallsignal mit einem demgegenüber höherfrequenten Audiosignal überlagert ist, und dass
mithilfe des Steuergerätes (4) die Frequenz und Amplitude sowie Zeitdauer des Körperschallsignals und des Audiosignals des betreffenden Körperschallwandlers (2) jeweils einstellbar

2. Einrichtung nach Anspruch 1, **dadurch gekennzeichnet, dass** die Körperschallwandler (2) gleichmäßig verteilt über die Grundplatte (1a, 1b) in ihrem Innern angeordnet sind.

3. Einrichtung nach Anspruch 1 oder 2, **dadurch gekennzeichnet, dass** die Grundplatte (1a, 1b) in zumindest zwei Segmente (6a, 6b) mit jeweils eigenen Körperschallwandlern (2) unterteilt ist.

4. Einrichtung nach Anspruch 3, **dadurch gekennzeichnet, dass** die Körperschallwandler (2) in den beiden Segmenten (6a, 6b) unterschiedlich angesteuert werden.

5. Einrichtung nach Anspruch 3 oder 4, **dadurch gekennzeichnet, dass** die beiden Segmente (6a, 6b) etwa gleich groß als beispielsweise Oberkörpersegment (6a) und Unterkörpersegment (6b) ausgebildet sind.

6. Einrichtung nach einem der Ansprüche 1 bis 5, **dadurch gekennzeichnet, dass** sich die Grundplatte (1a, 1b) aus zwei gleich großen Teilplatten (1a, 1b) zusammensetzt, die zwischen sich die Körperschallwandler (2) aufnehmen.

7. Einrichtung nach einem der Ansprüche 1 bis 6, **dadurch gekennzeichnet, dass** der jeweilige Körperschallwandler (2) mit seiner Montageplatte (2b) an eine Unterseite der oberen Teilplatte (1a) der Grundplatte (1a, 1b) angeschlossen ist.

8. Einrichtung nach Anspruch 7, **dadurch gekennzeichnet, dass** der jeweilige Körperschallwandler (2) mit seiner schwingfähigen Masse (2a) nach außen weisend auf der Oberseite der unteren Teilplatte (1b) angeordnet ist.

9. Einrichtung nach Anspruch 7 oder 8, **dadurch gekennzeichnet, dass** der an die obere Teilplatte (1a) angeschlossene jeweilige Körperschallwandler (2) mit einer textilen Flächenschicht (7) aus beispielsweise einer Watte oder einem Vlies, abgedeckt ist.

10. Einrichtung nach einem der Ansprüche 6 bis 9, **dadurch gekennzeichnet, dass** beide Teilplatten (1a, 1b) aus einem Schaumkunststoff, beispielsweise einer Stärke zwischen 2 cm und 5 cm hergestellt sind.

## Claims

1. Vibroacoustic device, in particular for treating humans with sound and/or vibrations, preferably a sound mat, with at least one flexible base plate (1a, 1b) and a sound generating unit (2, 4) having a plurality of structure-borne sound transducers (2), wherein
the respective structure-borne sound transducer (2) is embedded in the base plate (1a, 1b) in order to couple the generated structure-borne sound directly into at least one body part, wherein further
the sound generating unit (2, 4) includes at least one control unit (4), and wherein
in order to control the respective structure-borne sound transducer (2), the control unit (4) generates a constant and adjustable low-frequency structure-borne sound signal for direct coupling into the body part concerned, wherein
individual, selected structure-borne sound transducers (2) and/or groups of structure-borne sound transducer (2) can be controlled differently with the aid of the control unit (4),
**characterized in that**
the low-frequency structure-borne sound signal is overlaid with an audio signal at a higher frequency than said structure-borne sound signal, and that
the frequency and amplitude, and the duration of the structure-borne sound signal and of the audio signal of the structure-borne sound transducer (2) are each adjustable with the aid of the control unit (4).

2. Device according to Claim 1, **characterized in that** the structure-borne sound transducers (2) are arranged in a uniformly distributed manner in the interior of the base plate (1a, 1b).

3. Device according to Claim 1 or 2, **characterized in that** the base plate (1a, 1b) is divided into at least two segments (6a, 6b), each with its own structure-borne sound transducers (2).

4. Device according to Claim 3, **characterized in that** the structure-borne sound transducers (2) in the two segments (6a, 6b) are controlled differently.

5. Device according to Claim 3 or 4, **characterized in that** the two segments (6a, 6b) are approximately the same size and designed as, for example, an upper body segment (6a) and a lower body segment (6b).

6. Device according to any one of Claims 1 to 5, **characterized in that** the base plate (1a, 1b) is composed of two sub-plates (1a, 1b) of equal size, which accommodate the structure-borne sound transducers (2) between them.

7. Device according to any one of Claims 1 to 6, **characterized in that** the respective structure-borne sound transducer (2) is connected by its mounting plate (2b) to an underside of the upper sub-plate (1a) of the base plate (1a, 1b).

8. Device according to Claim 7, **characterized in that** the respective structure-borne sound transducer (2) is arranged on the upper side of the lower sub-plate (1b) with its vibratable mass (2a) facing outwards.

9. Device according to Claim 7 or 8, **characterized in that** the respective structure-borne sound transducer (2) connected to the upper sub-plate (1a) is covered with a textile surface layer (7) made of, for example, a batting or a nonwoven material.

10. Device according to any one of Claims 6 to 9, **characterized in that** both sub-plates (1a, 1b) are produced from a foam plastic, for example with a thickness between 2 cm and 5 cm.

## Revendications

1. Système vibro-acoustique destiné au traitement acoustique et/ou vibratoire humain, de préférence matelas sonore avec au moins une plaque de base souple (1a, 1b) ainsi qu'une unité génératrice de sons (2, 4) comportant plusieurs transducteurs acoustiques corporels (2), sachant que
le transducteur acoustique corporel (2) respectif est incorporé dans la plaque de base (1a, 1b) pour injecter directement le son corporel produit dans au moins une partie du corps,
sachant en plus que
l'unité génératrice de sons (2, 4) comporte au moins un appareil de commande (4), et
sachant que
l'appareil de commande (4) destiné à commander le transducteur acoustique corporel (2) respectif produit un signal acoustique corporel à basse fréquence constant et réglable pour l'injection directe dans la partie du corps concernée, sachant qu'à l'aide de l'appareil de commande (4) des transducteurs acoustiques corporels (2) sélectionnés individuels et/ou des groupes de transducteurs acoustiques corporels (2) peuvent être commandés de façon différente,
**caractérisé en ce que**
le signal acoustique corporel à basse fréquence est masqué par un signal audio de fréquence plus élevée en opposition et **en ce**
**qu'**à l'aide de l'appareil de commande (4), la fréquence et l'amplitude ainsi que la durée du signal acoustique corporel et du signal audio du transducteur acoustique corporel (2) concerné peuvent être respectivement réglées.

2. Système selon la revendication 1, **caractérisé en ce que** les transducteurs acoustiques corporels (2) sont disposés uniformément répartis dans leur intérieur sur la plaque de base (1a, 1b).

3. Système selon la revendication 1 ou 2, **caractérisé en ce que** la plaque de base (1a, 1b) est subdivisée en au moins deux segments (6a, 6b) avec respectivement des transducteurs acoustiques corporels propres (2).

4. Système selon la revendication 3, **caractérisé en ce que** les transducteurs acoustiques corporels (2) sont commandés différemment dans les deux segments (6a, 6b).

5. Système selon la revendication 3 ou 4, **caractérisé en ce que** les deux segments (6a, 6b) sont constitués à peu près de taille identique au segment corporel supérieur (6a) et au segment corporel inférieur (6b) par exemple.

6. Système selon l'une quelconque des revendications 1 à 5, **caractérisé en ce que** la plaque de base (1a, 1b) est composé de deux parties de plaque (1a, 1b) de même taille, qui reçoivent entre elles les transducteurs acoustiques corporels (2).

7. Système selon l'une quelconque des revendications 1 à 6, **caractérisé en ce que** le transducteur acoustique corporel (2) respectif est raccordé avec sa plaque de montage (2b) à une face inférieure de la partie de plaque supérieure (1a) de la plaque de base (1a, 1b).

8. Système selon la revendication 7, **caractérisé en ce que** le transducteur acoustique corporel respectif (2) est disposé avec sa masse pouvant osciller (2a) vers l'extérieur tourné sur le côté supérieur de la partie de plaque inférieure (1b).

9. Système selon la revendication 7 ou 8, **caractérisé en ce que** le transducteur acoustique corporel (2) respectif raccordé à la partie de plaque supérieure (1a) est couvert d'une couche superficielle textile (7) faite par exemple d'un coton hydrophile ou d'un non-tissé.

10. Système selon l'une quelconque des revendications 6 à 9, **caractérisé en ce que** les deux parties de plaque (1a, 1b) sont fabriquées dans une mousse de matière plastique, par exemple d'une épaisseur de 2 cm et 5 cm.
